# EUROPÄISCHE PATENTANMELDUNG

(11) **EP 1 674 427 A1**
(43) Veröffentlichungstag der Anmeldung: **28.06.2006**
(21) Anmeldenummer: 04030585.6
(22) Anmeldetag: 23.12.2004
(51) Int. Cl.: C01G 23/07, C01G 23/04, C09C 1/36

(54) **Strukturmodifizierte Titandioxide**

(71) Anmelder: Degussa AG, 40474 Düsseldorf (DE)
(72) Erfinder: Meyer, Jürgen, Dr., 63811 Stockstadt (DE); Hasenzahl, Steffen, Dr., Morris Plains, New Jersey, 07950 (US); Riedemann, Heike, 63776 Mömbris (DE)

(57) **Zusammenfassung**

Pyrogen hergestellte, strukturmodifizierte Titandioxide beziehungsweise pyrogen hergestellte, strukturmodifizierte Titandioxid-Mischoxide werden hergestellt, indem man die Titandioxide mit einer Kugelmühle behandelt und gegebenenfalls nachvermahlt.

Sie können in Sonnenschutzformulierungen eingesetzt werden.

## Beschreibung

Die Erfindung betrifft pyrogen hergestellte, strukturmodifizierte Titandioxide, beziehungsweise pyrogen hergestellte, strukturmodifizierte Titandioxid-Mischoxide, ein Verfahren zu ihrer Herstellung sowie ihre Verwendung in Sonnenschutzformulierungen.

Es ist bekannt, pyrogen hergestellte Titandioxide und pyrogen hergestellte Titandioxid-Eisenoxid Mischoxide in Sonnenschutzformulierungen einzusetzen (EP 0609533).

Die bekannten pyrogen hergestellten Titandioxide haben den Nachteil, daß sie und die damit hergestellten Sonnenschutzformulierungen bei dem Auftragen auf die Haut keine ausreichende Transparenz aufweisen.

Weiterhin ist bei der Vorbereitung eine aufwendige Dispergierung notwendig.

Eine starke Verdickungswirkung bei dem Dispergieren, in beispielsweise kosmetischen Ölen oder Wasser, erschwert die Herstellung von Dispersionen beziehungsweise Sonnenschutzmitteln mit einem hohen Titandioxidgehalt. Die mit den bekannten Titandioxiden hergestellten Sonnenschutzmittel erzeugen bei der Anwendung ein stumpfes Hautgefühl.

Die Aufgabe der Erfindung ist es, pyrogen hergestellte Titandioxide herzustellen, die in Sonnenschutzmitteln eine bessere Transparenz sowie ein besseres Hautgefühl aufweisen.

Gegenstand der Erfindung sind pyrogen hergestellte, strukturmodifizierte Titandioxide sowie pyrogen hergestellte, strukturmodifizierte Titandioxid-Mischoxide.

Ein weiterer Gegenstand der Erfindung ist ein Verfahren zur Herstellung der erfindungsgemäßen pyrogen hergestellten, strukturmodifizierten Titandioxide sowie pyrogen hergestellte, strukturmodifizierte Titandioxid-Mischoxide, welches dadurch gekennzeichnet ist, daß man das pyrogen hergestellte Titandioxid beziehungsweise pyrogen hergestelltes Titandioxid-Mischoxid mittels einer Kugelmühle behandelt und gegebenenfalls nachvermahlt.

Als Edukte beziehungsweise Ausgangsmaterial können beispielsweise die pyrogen hergestellten Titandioxide, die in der Tabelle 1 aufgeführt sind, eingesetzt werden.

**Tabelle 1**

| | Aeroxide® TiO₂ P25 | Aeroxide® TiO₂ P25 S | Aeroxide® TiO₂ PF2 |
|---|---|---|---|
| Aussehen | weißes Pulver | weißes Pulver | weißes Pulver |
| spez. Oberfläche (BET)¹⁾ m²/g | 50 ± 15 | 50 ± 15 | 57,5 ± 12,5 |
| mittl. Größe d. Primärteilchen nm | 21 | - | - |
| Stampfdichte (ca. Wert)²⁾ g/l | 130 | - | 80 |
| Schüttdichte (ca. Wert) g/l | - | 60 - 150 | - |
| Trockmmgsverlust³) (2 Std.105 °C) % | ≤ 1,5 | - | ≤ 2,0 |
| Glühverlust^{4) 7)} (2 Std. 1000 °C) % | ≤ 2,0 | - | ≤ 3,0 |
| pH-Wert⁵⁾ | 3,5 - 4,5 | - | 3,5 - 4,5 |
| As-Gehalt ppm | - | ≤ 1,0 | - |
| Hg-Gehalt ppm | - | ≤ 1,0 | - |
| Sb-Gehalt ppm | - | ≤ 2,0 | - |
| Pb-Gehalt ppm | - | ≤ 10 | - |
| SiO₂-Gehalt⁸⁾ Gew.-% | ≤ 0,200 | - | - |
| Al₂O₃-Gehalt⁸⁾ Gew.-% | ≤ 0,300 | - | - |
| Fe₂O₃-Gehalt⁸⁾ Gew.-% | ≤ 0,010 | - | 2,0 ± 1 |
| TiO₂-Gehalt⁸⁾ Gew.-% | ≥ 99,5 | ≥ 99,0; ≤ 100,5 | ≥ 94,00 |
| HCl-Gehalt¹⁰⁾ Gew.-% | ≤ 0,300 | ≤ 0,3 | ≤ 0,800 |
| Siebrückstand⁶⁾ Gew.-% (nach Mocker, 45µm) | ≤ 0,050 | - | - |

| | | | |
|---|---|---|---|
| ¹⁾ Nach DIN 66131 | | | |
| ²⁾ Nach DIN EN ISO 787-11, JIS K 5101/20 (ungesiebt) | | | |
| ³⁾ Nach DIN EN ISO 787-2, ASIM D280, JIS K 5101/23 | | | |
| ⁴⁾ Nach DIN EN ISO 3262-20, ASIM D 1208, JIS K 5101/24 | | | |
| ⁵⁾ Nach DIN EN ISO 787-9, ASIM D 1208, JIS K 5101/26 | | | |
| ⁶⁾ Nach DIN EN ISO 787-18, JIS K 5101/22 | | | |
| ⁷⁾ Basierend auf getrocknete Substanz (2 Std. bei 105 °C) | | | |
| ⁸⁾ Geglühte Substanz (2 Std. bei 1000 °C) | | | |
| ⁹⁾Spezielle feuchtigkeitsschützende Verpackung | | | |
| ¹⁰⁾ HCl-Gehalt ist Teil des Glühverlustes | | | |

Insbesondere kann als Edukt ein eisenoxidhaltiges Titandioxidpulver eingesetzt werden, welches aus dem Dokument EP 0 609 533 A1 bekannt ist.

Das eisenoxidhaltige Titandioxidpulver kann aus einem pyrogen hergestellten Eisenoxid-Titandioxid-Mischoxid mit einer BET-Oberfläche von 10 bis 150 m²/g, welches 0,5 bis 50 Gew.-% Eisenoxid enthält, bezogen auf die Gesamtmenge, als Bestandteil des Mischoxides bestehen.

Es kann hergestellt werden, indem man wasserfreies Eisen-(III)-chlorid verdampft, zusammen mit einem Inertgas, zum Beispiel Stickstoff, in die Mischkammer eines bekannten Brenners überführt, dort mit Wasserstoff, Luft und gasförmigen Titantetrachlorid in einem Verhältnis, das der Zusammensetzung des Eisenoxid-Titandioxid-Mischoxids entspricht, vermischt, das 4-Komponentengemisch in einer Reaktionskammer verbrennt, danach das feste Eisenoxid-Titandioxid-Mischoxid von den gasförmigen Reaktionsprodukten abtrennt und gegebenenfalls in feuchter Luft von anhaftendem Chlorwasserstoff befreit.

Die pyrogene Herstellung von Titandioxid P 25 ist bekannt aus Ullmann's Enzyklopädie der technischen Chemie Band 21, 4. Auflage (1982) Seite 464.

Das Titandioxid P 25 wird durch Flammenhydrolyse von Titantetrachlorid entsprechend der Gleichung

TiCl₄ + 2 H₂ + O₂ **→** TiO₂ + 4 HCl

hergestellt.

Das Titandioxid PF 2, welches ein mit 2% Eisenoxid dotiertes Titandioxid ist, wird ebenfalls nach diesem Verfahren entsprechend der Gleichung

TiCl₄ + 2 H₂ + O₂ **→** TiO₂ + 4 HCl

und

2 FeCl₃ + 3 H₂ + 1,5O₂ → Fe₂O₃ + 6 HCl

hergestellt.

Beide Produkte Titandioxid PF 2 und P 25 bestehen kristallographisch aus etwa 80 % Anatas und 20 % Rutil. Sie weisen eine mittlere Primärteilchengröße von ca. 20 nm auf.

Die physikalisch-chemischen Eigenschaften von Titandioxid PF 2 und P 25 sind in der Tabelle 1 zusammengefasst.

Das Titandioxid P 25 S weist die in der Tabelle 1 aufgeführten physikalisch-chemischen Eigenschaften auf.

Weiterhin kann als Edukt ein pyrogen hergestelltes Titandioxid (gemäß DE 103 57 508.1 eingesetzt werden, welches in Aggregaten von Primärpartikeln vorliegt, und dadurch gekennzeichnet ist, daß
- es eine BET-Oberfläche von 20 bis 200 m²/g und
- die Halbwertsbreite HB, in Nanometer, der Primärpartikelverteilung Werte zwischen
- HB [nm] = a x BET^{f} mit a = 670x10⁹ m³/g und
   -1,3 ≤ f ≤ -1,0 aufweist, und
- der Anteil von Partikeln mit einem Durchmesser von mehr als 45 µm in einem Bereich von 0,0001 bis 0,05 Gew.-% liegt.

Es kann hergestellt werden, indem man
- ein Titanhalogenid, bevorzugt Titantetrachlorid, bei Temperaturen von kleiner 200°C verdampft, die Dämpfe mittels eines Traggases mit einem Anteil an Wasserdampf in einem Bereich von 1 bis 25 g/m³ Traggas in eine Mischkammer überführt,
- separat Wasserstoff, Primärluft, die gegebenenfalls mit Sauerstoff angereichert und/oder vorerhitzt sein kann, und Wasserdampf in die Mischkammer überführt,
- wobei der Anteil an Wasserdampf in einem Bereich von 1 bis 25 g/m³ Primärluft liegt,
- der Lambda-Wert im Bereich von 1 bis 9 und der Gamma-Wert im Bereich von 1 bis 9 liegt,
   anschließend
- das Gemisch, bestehend aus dem Dampf des Titanhalogenides, Wasserstoff, Luft und Wasserdampf, in einem Brenner zündet und die Flamme in eine von der Umgebungsluft abgeschlossene Reaktionskammer hinein verbrennt, wobei
- in der Reaktionskammer ein Vakuum von 1 bis 200 mbar vorliegt, und die Austrittsgeschwindigkeit des Reaktionsgemisches aus der Mischkammer in den Reaktionsraum in einem Bereich von 10 bis 80 m/s liegen kann,
- in die Reaktionskammer zusätzlich Sekundärluft einbringt, wobei
- das Verhältnis Primärluft/Sekundärluft zwischen 10 und 0,5 liegen kann,
- anschließend den Feststoff von gasförmigen Stoffen abtrennt und
- nachfolgend den Feststoff mit Wasserdampf behandelt.

Die Strukturmodifizierung kann beispielsweise mit einer Kugelmühle oder einer kontinuierlich arbeitenden Kugelmühle erfolgen.

Die Nachvermahlung kann beispielsweise mittels einer Luftstrahlmühle, Zahnscheibenmühle oder Stiftmühle erfolgen.

Ein weiterer Gegenstand der Erfindung ist die Verwendung der erfindungsgemäßen pyrogen hergestellten, strukturmodifizierten Titandioxide zur Herstellung von Sonnenschutzmitteln beziehungsweise Lichtschutzformulierungen beziehungsweise Lichtschutzzubereitungen.

Ein weiterer Gegenstand der Erfindung sind Sonnenschutzformulierungen, welche dadurch gekennzeichnet sind, daß sie pyrogen hergestellte strukturmodifizierte Titandioxide beziehungsweise pyrogen hergestellte strukturmodifizierte Titandioxid-Mischoxide enthalten.

Die erfindungsgemäßen Sonnenschutzformulierungen der vorliegenden Erfindung können bevorzugt neben einer oder mehrerer Ölphasen zusätzlich eine oder mehrere Wasserphasen enthalten und beispielsweise in Form von W/O-, O/W-, W/O/W-oder O/W/O-Emulsionen vorliegen. Solche Formulierungen können vorzugsweise auch Mikroemulsionen, Stifte, Schäume (sog. Mousse), Feststoff-Emulsionen (d. h. Emulsionen, welche durch Feststoffe stabilisiert sind, z. B. Pickering-Emulsionen), sprühbare Emulsionen oder Hydrodispersionen sein. Des weiteren können die Zubereitungen vorteilhaft auch ölfreie und/oder wäßrige/alkoholische Lösungen sein.

Auch (makroskopisch) zwei- oder mehrphasige Systeme sind erfindungsgemäß vorteilhaft. "Zwei- oder mehrphasig" im Sinne der vorliegenden Erfindung bedeutet, daß zwei oder mehr Phasen separat übereinander geschichtet vorliegen. Es ist besonders vorteilhaft im Sinne der vorliegenden Erfindung, wenn mindestens eine der makroskopisch sichtbaren Phasen eine (W/O-, O/W-, Mikro-) Emulsion darstellt. Die Emulsion wird bei dieser (makroskopischen) Betrachtung als eine Phase wahrgenommen, obwohl es dem Fachmann natürlich bekannt ist, daß Emulsionen an sich aus zwei oder mehr miteinander homogenisierten Phasen gebildet werden. Die "Emulsionsphase" ist langzeitstabil, so daß es auch über einen längeren Zeitraum (Monate, Jahre) nicht zu einer Entmischung beziehungsweise Phasenauftrennung innerhalb der Emulsion kommt.

Die makroskopisch sichtbaren Phasen bzw. Schichten lassen sich vorteilhaft -zum Beispiel durch Schütteln - kurzfristig zu einer homogenen Emulsion emulgieren, welche aber nicht langzeitstabil ist, sondem sich vielmehr über einen Zeitraum von Minuten, Stunden oder Tagen wieder zu zwei oder mehr übereinander geschichteten Phasen entmischt.

Es ist besonders vorteilhaft im Sinne der vorliegenden Erfindung, wenn mindestens eine der makroskopisch sichtbaren Phasen eine Mikroemulsion und mindestens eine andere der makroskopisch sichtbaren Phasen eine Ölphase darstellt.

Besonders vorteilhaft im Sinne der vorliegenden Erfindung sind sprühbare O/W Emulsionen, insbesondere O/W-Mikroemulsionen.

Die Tröpfchendurchmesser der gewöhnlichen "einfachen", also nichtmultiplen Emulsionen liegen im Bereich von ca. 1 µm bis ca. 50 µm. Solche "Makroemulsionen" sind, ohne weitere färbende Zusätze, milchigweißgefärbt und opak. Feinere "Makroemulsionen", deren Tröpfchendurchmesser im Bereich von ca. 0,5 µm bis ca. 1 µm liegen, sind, wiederum ohne färbende Zusätze, bläulichweißgefärbt und opak. Solche "Makroemulsionen" haben für gewöhnlich ein hohe Viskosität.

Der Tröpfchendurchmesser von Mikroemulsionen im Sinne der vorliegenden Erfindung dagegen liegt im Bereich von etwa 50 bis etwa 500 nm. Derartige Mikroemulsionen sind bläulichweiß gefärbt bis transluzent und meist niedrigviskos. Die Viskosität vieler Mikroemulsionen vom O/W-Typ ist vergleichbar mit der des Wassers.

Vorteil von Mikroemulsionen ist, daß in der dispersen Phase Wirkstoffe wesentlich feiner dispers vorliegen können als in der dispersen Phase von "Makroemulsionen". Ein weiterer Vorteil ist, daß sie aufgrund ihrer niedrigen Viskosität versprühbar sind. Werden Mikroemulsionen als Kosmetika verwendet, zeichnen sich entsprechende Produkte durch hohe kosmetische Eleganz aus.

Erfindungsgemäß vorteilhaft sind insbesondere O/W-Mikroemulsionen, welche mit Hilfe der sogenannten Phaseninversionstemperatur-Technologie erhältlich sind und mindestens einen Emulgator (Emulgator A) enthalten, welcher gewählt wird aus der Gruppe der Emulgatoren mit folgenden Eigenschaften:
- ihre Lipophilie ist abhängig von der Temperatur, dergestalt daß durch Erhöhung der Temperatur die Lipophilie
zunimmt und durch Senkung der Temperatur die Lipophilie des Emulgators abnimmt.

Vorteilhafte Emulgatoren A sind z. B. polyethoxilierte Fettsäuren (PEG-100 Stearat,PEG-20 Stearat, PEG-150 Laurath, PEG-8 Distearat und dergleichen) und/oder polyethoxilierte Fettalkohole (Cetearath-12, Cetearath-20, lsoceteth-20, Beheneth-20, Laurath-9 etc.) und/oder Alkylpolyglycoside (Cetearyl Glycoside, Stearyl Glycoside, Palmity) Glycoside etc.).

Sofern die Phaseninversion im wesentlichen durch Variation der Temperatur eingeleitet wird, sind 0/W-Emulsionen, insbesondere 0/W-Mikroemulsionen erhältlich, wobei die Größe der Öltröpfchen im wesentlichen durch die Konzentration des oder der eingesetzten Emulgatoren bestimmt wird, dergestalt, daß eine höhere Emulgatorkonzentration kleinere Tröpfchen bewirkt und geringere Emulgatorkonzentration zu größeren Tröpfchen führt. Die Tröpfchengrößen liegen in der Regel zwischen 20 und 500 nm.

Es ist im Sinn der vorliegenden Erfindung gegebenenfalls vorteilhaft, weitere, nicht unter die Definition des Emulgators A fallende, W/O und/oder O/W-Emulgatoren zu verwenden, beispielsweise um die Wasserfestigkeit der Zubereitungen gemäß der vorliegenden Erfindung zu erhöhen.

Hier können z. B. Alkylmethiconcopolyole und/oder Alkyl-Dimethiconcopolyole (insbesondere Cetyl Dimethicone Copolyol, Lauryl Methicone Copolyol), W/O-Emulgatoren (wie z. B. Sorbitanstearat, Glycerylstearat, Glycerolstearat, Sorbitanoleat, Lecithin, Glycerylisostearat, Polyglyceryl-3-Oleat, Polyglyceryl-3 Diisostearat, PEG-7-hydriertes Ricinusöl, Polyglyceryl-4-Distearat, Acrylatl C10-3o-Alkylacrylat-Cresspolymer, Sorbitanisostearat, Poloxamer 101, Polyglyceryl-2-dipolyhydroxystearat, Polyglyceryl-3-Diisostearat, Polyglyceryl-4-dipolyhydroxystearat, PEG-30dipolyhydroxystearat, Diisostearoylpolyglyceryl-3-diisostearat, Glycol Distearat, Polyglyceryl-3dipolyhydroxystearat) und/oder Fettsäureester der Schwefeloder Phosphorsäure (Cetylphosphat, Trilaureth-4 Phosphat, Trioleth-8-Phosphat, Stearylphosphat, Cetearylsulfat etc.) verwendet werden.

Weitere vorteilhafte sprühbare 0/W-Emulsionen im Sinne der vorliegenden Erfindung sind dünnflüssige kosmetische oder dermatologische Hydrodispersionen, welche mindestens eine Ölphase und mindestens eine Wasserphase enthalten, wobei die Zubereitung durch mindestens einen Gelbildner stabilisiert wird und nicht notwendigerweise Emulgatoren enthalten muß, aber einen oder mehrere Emulgatoren enthalten kann.

Vorteilhafte Gelbildner für derartige Zubereitungen sind beispielsweise Copolymere aus Clo 3o-Alkylacrylaten und einem oder mehreren Monomeren der Acrylsäure, der Methacrylsäure oder deren Ester. Die INCI-Bezeichnung für solche Verbindungen ist "Acrylates/C1o-30Alkyl Acrylate Crosspolymer". Insbesondere vorteilhaft sind die Pemulen® Typen TR1, TR2 und TRZ von der Fa. Goodrich (Noveon). Auch Carbopole sind vorteilhafte Gelbildner für derartige Zubereitungen. Carbopole sind Polymere der Acrylsäure, insbesondere auch Acrylat-Alkylacrylat-Copolymere.

Vorteilhafte Carbopole sind beispielsweise die Typen 907, 910, 934, 940, 941, 951, 954, 980, 981, 1342, 1382, 2984 und 5984, ebenso die ETD-Typen 2020, 2050 und Carbopol Ultrez 10. Weitere vorteilhafte Gelbildner für derartige Zubereitungen sind Xanthan Gummi, Cellulose Derivate und Johannisbrotkernmehl.

Als mögliche (fakultative) Emulgatoren können ethoxilierte Fettalkohole oder ethoxilierte Fettsäuren (insbesondere PEG-100 Stearat, Ceteareth-20) und/oder andere nichtionische oberflächenaktive Substanzen verwendet werden.
Ferner vorteilhaft können die sehr dünnflüssigen bis sprühbaren Emulsionen auch W/O- bzw. Wasser-in-Silikonöl-(W/S-) Emulsionen sein. Insbesondere vorteilhaft sind W/O- bzw. W/S-Emulsionen, die
- mindestens einen Silikonemulgator (W/S) mit einem HLB-Wert ≤ 8 und/oder mindestens einen W/O-Emulgator mit einem HLB-Wert < 7 und
mindestens einen O/W-Emulgator mit einem HLB-Wert > 10 enthalten.

Derartige Zubereitungen enthalten ferner mindestens 20 Gew.-% Lipide, wobei die Lipidphase vorteilhaft auch Silikonöle enthalten bzw. sogar ganz aus solchen bestehen kann. Der oder die Silikonemulgatoren kann vorteilhaft aus der Gruppe der Alkylmethiconcopolyole und/oder Alkyl-Dimethiconcopolyole gewählt werden (z.B. Dimethiconcopoiyole, welche von der Goldschmidt AG unter den Warenbezeichnungen ABIL® B 8842, ABIL® B 8843, ABIL® B 8847, ABIL® B 8851, ABIL® B 8852, ABIL® B 8863, ABIL® B 8873 und ABIL® B 88183 verkauft werden, Cetyl Dimethiconcopolyol [Goldschmidt AG/ABIL® EM 90], Cyclomethicon Dimethiconcopolyol [Goldschmidt AG/ABIL® EM 97], Laurylmethiconcopolvol [Dow Corning Ltd. I Dow Corninge 5200 Formulation Aid], Octyl Dimethicon Ethoxy Glucosid [Firma Wacker].

Der oder die W/O-Emulgatoren mit einem HLB-Wert < 7 kann vorteilhaft aus folgender Gruppe gewählt werden:
Sorbitanstearat, Sorbitanoleat, Lecithin, Glyceryllanolat, Lanolin, hydriertem Ricinusöl, Glycerylisostearat, Polyglyceryl-3-Oleat, Pentaerythrithylisostearat, Methylglucosedioleat, Methylglucosedioleat im Gemisch mit Hydroxystearat und Bienenwachs, PEG-7-hydriertes Ricinusöl, Polyglyceryl-4-isostearat, Hexyllaurat, Acrylat/ C₁ₒ₋₃ₒ₋Alkylacrylat-Crosspolymer, Sorbitanisostearat, Polyglyceryl-2-dipolyhydroxystearat, Polyglyceryl-3-Diisostearat, PEG-30-dipolyhydroxystearat, Diisostearoylpolyglyceryl-3-diisostearat, Polyglyceryl-3dipolyhydroxystearat, Polyglyceryl-4-dipolyhydroxystearat, Polyglyceryl-3-dioleat.

Der oder die O/W-Emulgatoren mit einem HLB-Wert > 10 kann vorteilhaft aus folgender Gruppe gewählt werden:
Glycerylstearat im Gemisch mit Ceteareth-20, Ceteareth-25, Ceteareth-6 im Gemisch mit Stearylalkohol, Cetylstearylalkohol im Gemisch mit PEG-40-Ricinusöl und Natriumcetylstearylsulfat, Triceteareth-4 Phosphat, Glycerylstearat, Natriumcetylstearylsulfat, Lecithin Trilaureth-4 Phosphat, Laureth-4 Phosphat, Stearinsäure, Propylenglycolstearat SE, PEG-9-Stearat, PEG-20 Stearat, PEG-30-Stearat, PEG-40-Stearat, PEG-100-Stearat, Ceteth-2, Ceteth-20, Polysorbate-20, Polysorbate-60, Polysorbate-65, Polysorbate-100, Glycerylstearat im Gemisch mit PEG-100 Stearat, Ceteareth-3, lsostearylglycerylether, Cetylstearylalkohol im Gemisch mit Natrium Cetylstearylsulfat, PEG-40-Stearat, Glycol Distearat, Polyglyceryl-2-PEG-4-Stearat, Ceteareth-12, Ceteareth-20, Ceteareth-30, Methylglucosesesquistearat, Steareth-10, PEG-20-Stearat, Steareth-21, Steareth-20, Isosteareth-20, PEG-45/Dodecylglycol-Copolymer, Glycerylstearat SE, Ceteth-20, PEG-20-Methylglucosesesquistearat, Glycerylstearatcitrat, Cetylphosphat, Cetearyl Sulfat, Sorbitansesquioleat, Triceteareth-4-Phosphat, Trilaureth-4-Phosphat, Polyglyceryl-methylglucosedistearat, Kaliumcetylphosphat, Isosteareth-10, Polyglyceryl-2-sesquiisostearat, Ceteth-10, Isoceteth-20, Glycerylstearat im Gemisch mit Ceteareth-20, Ceteareth-12, Cetylstearylalkohol und Cetylpalmitat, PEG-30-Stearat, PEG-40-Stearat, PEG-100-Stearat.

Ferner vorteilhaft sind wäßrig-alkoholische Lösungen. Sie können von 0 Gew.-% bis 90 Gew.-% Ethanol enthalten. Wäßrig-alkoholische Lösungen können im Sinne der vorliegenden Erfindung vorteilhaft auch Lösungsvermittler enthalten, wie z.B. PEG-40 oder PEG-60 hydrogeniertes Ricinusöl.

Die Zubereitungen gemäß der vorliegenden Erfindung können vorteilhaft auch als kosmetische oder dermatologische Tränkungslösungen, mit welchen insbesondere wasserunlösliche Substrate - wie z.B. gewebte oder nichtgewebte Tücher - befeuchtet sind, verwendet werden. Derartige Tränkungslösungen sind vorzugsweise dünnflüssig, insbesondere sprühbar (wie z.B. PIT Emulsionen, Hydrodispersionen, W/O-Emulsionen, Öle, wäßrige Lösungen etc.) und haben vorzugsweise eine Viskosität von weniger als 2000 mPa s, insbesondere weniger als 1.500 mPa s (Meßgerät: Haake Viskotester VT 02 bei 25°C). Mit ihrer Hilfe sind beispielsweise kosmetische Sonnenschutztücher, Pflegetücher und dergleichen erhältlich, welche die Kombination eines weichen, wasserunlöslichen Materials mit der dünnflüssigen kosmetischen und dermatologischen Tränkungslösung darstellen.

Die Zubereitungen gemäß der vorliegenden Erfindung können vorteilhaft auch als wasserfreie Öle oder Ölgele oder Pasten vorliegen. Vorteilhafte Öle sind z.B. synthetische, halbsynthetische oder natürliche Öle wie beispielsweise Rapsöl, Reisöl, Avocadoöl, Olivenöl, Mineralöl, Cocoglyceride, Butylene Gylcol Dicaprylat1Dicaprat, C₁₂-C₁₅ Alkylbenzoat, Dicaprylyl Carbonat, Octyldodekanol und dergleichen mehr. Als Ölgelbildner können verschiedenste Wachse mit einem Schmelzpunkt > 25 c verwendet werden. Ferner vorteilhaft sind Gelbildner aus der Gruppe der Aerosile, der Alkylgalaktomannane (z.B. N-Hance AG 200 und N-Hance AG 50 von der Fa. Hercules) und Polyethylene-Derivate.

Besonders vorteilhaft im Sinne der vorliegenden Erfindung sind ferner selbstschäumende, schaumförmige, nachschäumende oder schäumbare kosmetische und dermatologische Zubereitungen.

Unter "selbstschäumend", "schaumförmig", "nachschäumend" bzw. "schäumbar" sind Zubereitungen zu verstehen, aus welchen Schäume - sei es bereits während des Herstellprozesses, sei es bei der Anwendung durch den Verbraucher oder auf andere Weise - durch Eintrag eines oder mehrerer Gase im Prinzip herstellbar sind. In derartigen Schäumen liegen die Gasbläschen (beliebig) verteilt in einer (oder mehreren) flüssigen Phase(n) vor, wobei die (aufgeschäumten) Zubereitungen makroskopisch nicht notwendigerweise das Aussehen eines Schaumes haben müssen. Erfindungsgemäße (aufgeschäumte) kosmetische oder dermatologische Zubereitungen (im folgenden der Einfachheit halber auch als Schäume bezeichnet) können z.B. makroskopisch sichtbar dispergierte Systeme aus in Flüssigkeiten dispergierten Gasen darstellen. Der Schaumcharakter kann aber beispielsweise auch erst unter einem (Licht-) Mikroskop sichtbar werden.

Darüber hinaus sind erfindungsgemäße Schäume - insbesondere dann, wenn die Gasbläschen zu klein sind, um unter einem Lichtmikroskop erkannt zu werden - auch an der starken Volumenzunahme des Systems erkennbar.

Es war insbesondere überraschend und beruht ebenfalls auf erfinderischer Tätigkeit, daß durch die Verwendung der erfindungsgemäßen Alpha Olefin/Maleinsäureanhydrid-Copolymere der Eintrag von Gasen unterstützt sowie über eine längere Lagerdauer auch bei höheren Temperaturen (z.B. 40 °C ) ein stabilisierender sowie deutlich schaumsteigernder Effekt erzielt werden kann. Es war dabei insbesondere erstaunlich, daß auf die Verwendung besonderer Tenside verzichtet werden kann. Der Eintrag von Gasen ist gegenüber dem Stand der Technik überraschenderweise außerordentlich erhöht. So kann beispielsweise eine Schaumverstärkung mit bis zu 100%ig erhöhtem Gasvolumen erzielt werden, ohne nach dem Stand der Technik übliche Schäummittel wie Tenside zu verwenden.

Hierdurch ist es möglich, Rezepturen mit hohem Gasvolumen (Luft und/oder andere Gase wie Sauerstoff, Kohlendioxid, Stickstoff, Helium, Argon u.a.) über lange Lagerdauer bei hohen Temperaturen stabil zu generieren.

Gegenstand der Erfindung ist daher ferner die Verwendung eines oder mehrerer Alpha Olefin/Maleinsäureanhydrid-Copolymere zur Schaumverstärkung selbstschäumender, schaumförmiger, nachschäumender oder schäumbarer kosmetischer und dermatologischer Zubereitungen.

Unter "Schaumverstärkung" ist im Sinne der vorliegenden Erfindung zu verstehen, daß der Eintrag von Gasen in die erfindungsgemäßen Schäume gegenüber dem Eintrag in ansonsten gleiche Zubereitungen, welche keine erfindungsgemäßen Alpha Olefin/Maleinsäureanhydrid-Copolymere enthalten, außerordentlich erhöht ist. Die erfindungsgemäßen Schäume können dementsprechend ein deutlich höheres Gasvolumen aufnehmen als Zubereitungen, welche keine erfindungsgemäßen Alpha Olefin/Maleinsäureanhydrid-Copolymere enthalten.

Mit "Schaumverstärkung" ist darüberhinaus gemeint, daß die Stabilität der aufgeschäumten Zubereitungen (die "Schaumstabilität") gegenüber ansonsten gleichen Zubereitungen, welche keine erfindungsgemäßen Alpha Olefin/Maleinsäureanhydrid-Copolymere enthalten, deutlich verbessert wird, d.h. durch die erfindungsgemäße Verwendung wird ein Brechen der Schäume zeitlich verzögert.

Derartige Zubereitungen enthalten im Sinne der vorliegenden Erfindung vorteilhaft ein Emulgatorsystem, welches aus
A) mindestens einem Emulgator A, gewählt aus der Gruppe der ganz-, teil- oder nicht neutralisierten, verzweigten und/oder unverzweigten, gesättigten und/oder ungesättigten Fettsäuren mit einer Kettenlänge von 10 bis 40 Kohlenstoffatomen,
B) mindestens einem Emulgator B, gewählt aus der Gruppe der polyethoxylierten Fettsäurester mit einer Kettenlänge von 10 bis 40 Kohlenstoffatomen und mit einem Ethoxylierungsgrad von 5 bis 100 und
C) mindestens einem Coemulgator C, gewählt aus der Gruppe der gesättigten und/oder ungesättigten, verzweigten und/oder unverzweigten Fettalkohole mit einer Kettenlänge von 10 bis 40 Kohlenstoffatomen besteht.

Der oder die Emulgatoren A werden vorzugsweise gewählt aus der Gruppe der Fettsäuren, welche ganz oder teilweise mit üblichen Alkalien (wie z.B. Natrium- und/oder Kaliumhydroxid, Natrium- und/oder Kaliumcarbonat sowie Mono- und/oder Triethanolainin) neutralisiert sind. Besonders vorteilhaft sind beispielsweise Stearinsäure und Stearate, Isostearinsäure und Isostearate, Palmitinsäure und Palmitate sowie Myristinsäure und Myristate.

Der oder die Emulgatoren B werden vorzugsweise gewählt aus der folgenden Gruppe: PEG-9-Stearat, PEG-8 Distearat, PEG-20-Stearat, PEG-8-Stearat, PEG-8-Oleat, PEG-25-Glyceryltrioleat, PEG-40-Sorbitanlanolat, PEG-15-Glycerylricinoleat, PEG-20-Glycervlstearat, PEG-20-Glycerylisostearat, PEG-20-Glvceryloleat, PEG-20-Stearat, PEG-20-Methylglucosesesquistearat, PEG-30-Glycerylisostearat, PEG-20-Glyceryllaurat, PEG-30-Stearat, PEG-30-Glycerylstearat, PEG-40-Stearat, PEG-30-Glyceryllaurat, PEG-50-Stearat, PEG-100-Stearat, PEG-150-Laurat. Besonders vorteilhaft sind beispielsweise polvethoxylierte Stearinsäurcester.

Der oder die Coemulgatoren C werden erfindungsgemäß vorzugsweise aus der folgenden Gruppe gewählt:
Behenylalkohol (C₂₂H₄₅0H) , Cetearylalkohol [eine Mischung aus Cetylalkohol (C₁₆H₃₃0H) und Stearylalkohol (C₁₈H₃₇0H)], Lanolinalkohole (Wollwachsalkohole, die die unverseifbare Alkoholfraktion des Wollwachses darstellen, die nach der Verseifung von Wollwachs erhalten wird). Besonders bevorzugt sind Cetyl- und Cetylstearylalkohol.

Es ist erfindungsgemäß vorteilhaft, die Gewichtsverhältnisse von Emulgator A zu Emulgator B zu Coemulgator C (A: B : C) wie a : b : c zu wählen, wobei a, b und c unabhängig voneinander rationale Zahlen von 1 bis 5, bevorzugt von 1 bis 3 darstellen können. Insbesondere bevorzugt ist ein Gewichtsverhältnis von etwa 1 : 1 : 1.

Es ist vorteilhaft im, Sinne der vorliegenden Erfindung, die Gesamtmenge der Emulgatoren A und B und des Coemulgators C aus dem Bereich von 2 bis 20 Gew.-%, vorteilhaft von 5 bis 15 Gew.-%, insbesondere von 7 bis 13 Gew.-%, jeweils bezogen auf das Gesamtgewicht der Formulierung, zu wählen.

Besonders vorteilhaft im Sinne der vorliegenden Erfindung sind ferner kosmetische oder dermatologische Zubereitungen, welche nur durch feinstverteilte Feststoffteilchen stabilisiert sind. Solche "emulgatorfreien" Emulsionen werden auch als Pickering-Emulsionen bezeichnet.

In Pickering-Emulsionen kommt es zu einer Anreicherung des festen Stoffes an der Phasengrenze ÖI/Wasser in Form einer Schicht, wodurch ein Zusammenfließen der dispersen Phasen verhindert wird. Von wesentlicher Bedeutung sind dabei insbesondere die Oberflächeneigenschaften der Feststoffpartikel, welche sowohl hydrophile als auch lipophile Eigenschaften zeigen sollten.

Vorteilhaft können die stabilisierenden Feststoffteilchen auch oberflächlich wasserabweisend behandelt ("gecoatet") sein, wobei ein amphiphiler Charakter dieser Feststoffteilchen gebildet werden beziehungsweise erhalten bleiben soll. Die Oberflächenbehandlung kann darin bestehen, daß die Feststoffteilchen nach an sich bekannten Verfahren mit einer dünnen hydrophoben bzw. hydrophilen Schicht versehen werden.

Der mittlere Partikeldurchmesser der als Stabilisator verwendeten mikrofeinen Feststoffteilchen wird vorzugsweise kleiner als 100 µm, besonders vorteilhaft kleiner als 50 µm gewählt. Dabei ist es im wesentlichen unerheblich, in welcher Form (Plättchen, Stäbchen, Kügelchen etc.) beziehungsweise Modifikation die verwendeten Feststoffteilchen vorliegen.

Vorzugsweise werden die mikrofeinen Feststoffteilchen aus der Gruppe der amphiphilen Metalloxidpigmente gewählt. Vorteilhaft sind insbesondere:
- Titandioxide (gecoatet und ungecoatet): z.B. Eusolex T-2000 von der Fa. Merck, Titandioxid MT 100 Z von der Tayca Corporation
- Zinkoxide z.B. Z-Cote und Z-Cote HP1 von der BASF AG, MZ-300, MZ-500 und MZ-505M von der Fa. Tayca Corporation
- Eisenoxide

Des weiteren ist es vorteilhaft, wenn die mikrofeinen Feststoffteilchen aus der folgenden Gruppe gewählt werden:
Bornitride, Stärkederivate (Tapioca Starch, Sodium Corn Starch Octynylsuccinat etc.), Talkum, Latexpartikel.

Es ist erfindungsgemäss vorteilhaft, wenn die feststoffstabilisierten Emulsionen deutlich weniger als 0,5 Gew.-% eines oder mehrerer Emulgatoren enthalten beziehungsweise sogar gänzlich emulgatorfrei sind.

Weiterhin vorteilhaft im Sinne der Erfindung sind Zubereitungen in Form von Stiften. Technisch betrachtet sind die meisten Stiftformulierungen wasserfreie Fettmischungen aus festen oder halbfesten Wachsen und flüssigen Ölen, wobei hochgereinigte Paraffinöle und - wachse die Stiftgrundmasse darstellen.

Übliche Grundstoffe für stiftförmige Zubereitungen sind beispielsweise flüssige Öle (wie z.B. Paraffinöle, Ricinusöl, Isopropylmyristat, C₁₂₋₁₅ Alkylbenzoat), halbfeste Bestandteile (z.B. Vaseline, Lanolin), feste Bestandteile (z.B. Bienenwachs, Ceresin und mikrokristalline Wachse beziehungsweise Ozokerit) und/oder hochschmelzende Wachse (z.B. Carnaubawachs, Candelillawachs). Auch wasserhaltige stiftförmige Zubereitungen sind an sich bekannt, wobei diese auch in Form von W/O-Emulsionen vorliegen können.

Die erfindungsgemässen kosmetischen oder dermatologischen Lichtschutzformulierungen können wie üblich zusammengesetzt sein und dem kosmetischen oder dermatologischen Lichtschutz, ferner zur Behandlung, Pflege und Reinigung der Haut und/oder der Haare und als Schminkprodukt in der dekorativen Kosmetik dienen.

Entsprechend ihrem Aufbau können kosmetische oder topische dermatologische Zusammensetzungen im Sinne der vorliegenden Erfindung, beispielsweise verwendet werden als Hautschutzcrème, Reinigungsmilch, Tages- oder Nachtcreme usw. Es ist gegebenenfalls möglich und vorteilhaft, die erfindungsgemässen Zusammensetzungen als Grundlage für pharmazeutische Formulierungen zu verwenden.

Zur Anwendung werden die kosmetischen und dermatologischen Zubereitungen in der für Kosmetika üblichen Weise auf die Haut und/oder die Haare in ausreichender Menge aufgebracht.

Die kosmetischen und dermatologischen Zubereitungen gemäß der Erfindung können kosmetische Hilfsstoffe enthalten, wie sie üblicherweise in solchen Zubereitungen verwendet werden, z.B. Konservierungsmittel, Konservierungshelfer, Komplexbildner, Bakterizide, Parfüme, Substanzen zum Verhindern oder Steigern des Schäumens, Farbstoffe, Pigmente, die eine färbende Wirkung haben, Verdickungsmittel, anfeuchtende und/oder feuchhaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Vorteilhafte Konservierungsmittel im Sinne der vorliegenden Erfindung sind beispielsweise Formaldehydabspalter (wie z.B. DMDM Hydantoin, welches beispielsweise unter der Handelsbezeichnung Glydant TM von der Fa. Lonza erhältlich ist), Iodopropylbutylcarbamate (z.B. die unter den Handelsbezeichnungen Glycacil-L, Glycacil-S von der Fa. Lonza erhältlichen und/oder Dekaben LMB von Jan Dekker), Parabene (d.h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäss ferner vorteilhaft auch Konservierungshelfer, wie beispielsweise Octoxyglycerin, Glycine Soja etc.

Vorteilhafte Komplexbildner im Sinne der vorliegenden Erfindung sind beispielsweise EDTA, [S,S]-Ethylendiamindisuccinat (EDDS), welches beispielsweise unter der Handelsbezeichnung Octaquest von der Fa. Octel erhältlich ist, Pentanatrium-Ethylendiamintetramethylenphosphonat, welches z.B. unter dem Handelsnamen Dequest 2046 von der Fa. Monsanto erhältlich ist und/oder Iminodibersteinsäure, welche u.a. von der Fa. Bayer AG unter den Handelsnamen Iminodisuccinat VP OC 370 (ca. 30%ige Lösung) und Baypure CX 100 fest erhältlich ist.

Besonders vorteilhafte Zubereitungen werden ferner erhalten, wenn als Zusatz- oder Wirkstoffe Antioxidantien eingesetzt werden. Erfindungsgemäss enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z.B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A beziehungsweise Vitamin-A-Derivate, beziehungsweise Carotine beziehungsweise deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die kosmetischen Zubereitungen gemäss der vorliegenden Erfindung kosmetische oder dermatologische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z.B. alpha-Liponsäure, Phytoen, D-Biotin, Coenzym Q10, alpha-Glucosylrutin, Carnitin, Carnosin, natürliche und/oder synthetische Isoflavonoide, Kreatin, Taurin und/oder beta -Alanin sowie 8-Hexadecen-1,16-dicarbonsäure (Dioic acid, CAS-Nummer 20701-68-2; vorläufige INCI-Bezeichnung Octadecendioic acid).

Erfindungsgemässe Rezepturen, welche z.B. bekannte Antifaltenwirkstoffe wie Flavonglycoside (insbesondere alpha -Glycosylrutin), Coenzym Q10, Vitamin E und/oder Derivate und dergleichen enthalten, eignen sich insbesondere vorteilhaft zur Prophylaxe und Behandlung kosmetischer oder dermatologischer Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefässerweiterungen (Teleangiektasien, Cuperosis), Schlaffheit und Ausbildung von Falten und Fältchen, lokale Hyper-, Hypo- und Fehlpigmentierungen (z.B. Altersflecken), vergrösserte Anfälligkeit gegenüber mechanischem Stress (z.B. Rissigkeit) und dergleichen). Weiterhin vorteilhaft eignen sie sich gegen das Erscheinungsbild der trockenen beziehungsweise rauhen Haut.

Die Wasserphase der Zubereitungen gemäß der vorliegenden Erfindung kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Butylenglykol, Ethylenglykol, Ethylenglykolmonoethyl- oder -monobutylether, Propylenglykolmonomethyl, -monoethyl- oder -monobutylether, Diethylenglykolmonomethyl- oder - monoethylether und analoge Produkte, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Polysaccharide beziehungsweise deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole [von der Fa. Bf. Goodrich], beispielsweise Carbopole der Typen 980, 981, 1382, 2984, 5984, ETD 2020, ETD 2050, Ultrez 10, jeweils einzeln oder in Kombination.

Die Zubereitungen gemäss der vorliegenden Erfindung können ferner vorteilhaft auch Selbstbräunungssubstanzen enthalten, wie beispielsweise Dihydroxyacteon und/oder Melaninderivate in Konzentrationen von 1 Gew.-% bis zu 8 Gew.-%, bezogen auf das Gesamtgewicht der Zubereitung.

Ferner vorteilhaft können die Zubereitungen gemäß der vorliegenden Erfindung auch Repellentien zum Schutz vor Mücken, Zecken und Spinnen und dergleichen enthalten. Vorteilhaft sind z.B. N,N-Diethyl-3-methylbenzamid (Handelsbezeichnung: Metadelphene, "DEET"), Dimethylphtalat (Handelsbezeichnung: Palatinol M, DMP) sowie insbesondere 3-(N-n-Butyl-N-acetyl-amino)-propionsäureethylester (unter dem Handelsnamen Insekt Repellent TM 3535 bei der Fa. Merck erhältlich). Die Repellentien können sowohl einzeln als auch in Kombination eingesetzt werden.

Als Moisturizer werden Stoffe oder Stoffgemische bezeichnet, welche kosmetischen oder dermatologischen Zubereitungen die Eigenschaft verleihen, nach dem Auftragen beziehungsweise Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel TM 1000 von der Gesellschaft SOLABIA S. A. erhältlich ist. Moisturizer können vorteilhaft auch als Antifaltenwirkstoffe zur Prophylaxe und Behandlung kosmetischer oder dermatologischer Hautveränderungen, wie sie z. B. bei der Hautalterung auftreten, verwendet werden.

Die erfindungsgemässen kosmetischen oder dermatologischen Zubereitungen können ferner vorteilhaft, wenngleich nicht zwingend, Füllstoffe enthalten, welche z.B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z.B. Tapiocastärke, Distärkephosphat, Aluminiumbeziehungsweise Natrium-Stärke Octenylsuccinat und dergleichen), Pigmente, die weder hauptsächlich UV-Filternoch färbende Wirkung haben (wie z.B. Bornitrid etc.) und/oder Aerosil® .

Die Ölphase der erfindungsgemässen Formulierungen wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z.B. Cocoglycerid, Olivenöl, Sonnenblumenöl, Sojaöl, Erdnussöl, Rapsöl, Mandelöl, Palmöl, Kokosöl, Rizinusöl, Weizenkeimöl, Traubenkernöl, Distelöl, Nachtkerzenöl, Macadamianussöl und dergleichen mehr.

Erfindungsgemäss vorteilhaft sind ferner z.B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z.B. Jojobaöl.

Ferner kann die Ölphase vorteilhaft gewählt werden aus der Gruppe der Dialkylether und Dialkylcarbonate, vorteilhaft sind z.B. Dicaprylylether (Cetiol OE) und/oder Dicaprylylcarbonat, beispielsweise das unter der Handelsbezeichnung Cetiol CC bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃₋Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemässen Formulierungen einen Gehalt an C₁₂₋₁₅-Alkylbenzoat aufweist oder vollständig aus diesem besteht.

Vorteilhafte Ölkomponenten sind ferner z.B. Butyloctylsalicylat (beispielsweise das unter der Handelsbezeichnung Hallbrite BHB bei der Fa. CP Hall erhältliche), Hexadecylbenzoat und Butyloctylbenzoat und Gemische davon (Hallstar AB) und/oder Diethylhexylnaphthalat (Hallbrite TQ oder Corapan TQ von H&R).

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan.

Unter den Polyolefinen sind Polydecene die bevorzugten Substanzen.

Vorteilhaft kann die Ölphase ferner einen Gehalt an cyclischen oder linearen Silikonölen aufweisen oder vollständig aus solchen Ölen bestehen, wobei allerdings bevorzugt wird, ausser dem Silikonöl oder den Silikonölen einen zusätzlichen Gehalt an anderen Ölphasenkomponenten zu verwenden.

Silikonöle sind hochmolekulare synthetische polymere Verbindungen, in denen Silicium- Atome über SauerstoffAtome ketten- und/oder netzartig verknüpft und die restlichen Valenzen des Siliciums durch KohlenwasserstoffReste (meist Methyl-, seltener Ethyl-, Propyl-, PhenylGruppen u.a.) abgesättigt sind. Systematisch werden die Silikonöle als Polyorganosiloxane bezeichnet. Die methylsubstituierten Polyorganosiloxane, welche die mengenmässig bedeutendsten Verbindungen dieser Gruppe darstellen und sich durch die folgende Strukturformel auszeichnen 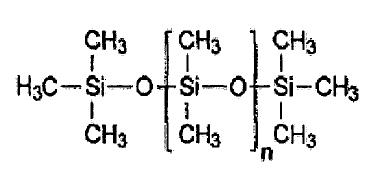 werden auch als Polydimethylsiloxan bzw. Dimethicon (INCI) bezeichnet. Dimethicone gibt es in verschiedenen Kettenlängen bzw. mit verschiedenen Molekulargewichten.

Besonders vorteilhafte Polyorganosiloxane im Sinne der vorliegenden Erfindung sind beispielsweise Dimethylpolysiloxane [Poly(dimethylsiloxan)], welche beispielsweise unter den Handelsbezeichnungen Abil 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silikone (Octamethylcyclotetrasiloxan beziehungsweise Decamethylcyclopentasiloxan), welche nach INCI auch als Cyclomethicone bezeichnet werden, aminomodifizierte Silikone (INCI: Amodimethicone) und Silikonwachse, z.B. Polysiloxan-Polyalkylen- Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Die Zubereitungen gemäss der vorliegenden Erfindung können ferner vorteilhaft eine oder mehrere Substanzen aus der folgenden Gruppe der Siloxanelastomere enthalten, beispielsweise um die Wasserfestigkeit und/oder den Lichtschutzfaktor der Produkte zu steigern:
a) Siloxanelastomere, welche die Einheiten R₂SiO und RSiO_{1,5} und/oder R₃SiO_{0,5} und/oder SiO₂ enthalten, wobei die einzelnen Reste R jeweils unabhängig voneinander Wasserstoff, C₁₋₂₄-Alkyl (wie beispielsweise Methyl, Ethyl, Propyl) oder Aryl (wie beispielsweise Phenyl oder Tolyl), Alkenyl (wie beispielsweise Vinyl) bedeuten und das Gewichtsverhältnis der Einheiten R2SiO zu RSiO_{1,5} aus dem Bereich von 1 : 1 bis 30 : 1 gewählt wird;
b) Siloxanelastomere, welche in Silikonöl unlöslich und quellfähig sind, die durch die Additionsreaktion eines Organopolysiloxans (1), das siliciumbebundenen Wasserstoff enthält, mit einem Organopolysiloxan (2), das ungesättigte aliphatische Gruppen enthält, erhältlich sind, wobei die verwendeten Mengenanteile so gewählt werden, daß die Menge des Wasserstoffes des Organopolysiloxans (1) oder der ungesättigten aliphatischen Gruppen des Organopolysiloxans (2)
   - im Bereich von 1 bis 20 mol-% liegt, wenn das Organopolysiloxan nicht cyclisch ist und
   - im Bereich von 1 bis 50 mol-% liegt, wenn das Organopolysiloxan cyclisch ist.

Vorteilhaft im Sinne der vorliegenden Erfindung liegen das oder die Siloxanelastomere in Form sphärischer Puder oder in Form von Gelen vor. Erfindungsgemäss vorteilhafte in Form sphärischer Puder vorliegende Siloxanelastomere sind die mit der INCI-Bezeichnung Dimethicone/Vinyl Dimethicone Crosspolymer, beispielsweise das von DOW CORNING unter der Handelsbezeichnungen DOW CORNING 9506 Powder erhältliche.

Besonders bevorzugt ist es, wenn das Siloxanelastomer in Kombination mit Ölen aus Kohlenwasserstoffen tierischer und/oder pflanzlicher Herkunft, synthetischen Ölen, synthetischen Estern, synthetischen Ethern oder deren Gemischen verwendet wird.

Ganz besonders bevorzugt ist es, wenn das Siloxanelastomer in Kombination mit unverzweigten bei Raumtemperatur flüssigen oder pastösen Silikonölen oder cyclischen Silikonölen oder deren Gemischen verwendet wird. Insbesondere vorteilhaft sind Organopolysiloxanelastomere mit der INCI-Bezeichnung Dimethicone/Polysilicone-11, ganz besonders die von der Grant Industries Inc. erhältlichen Gransil-Typen GCM, GCM-5, DMG-6, CSE Gel, PM-Gel, LTX, ININ Gel, AM-18 Gel und/oder DMCM-5.

Ganz aussergewöhnlich bevorzugt ist es, wenn das Siloxanelastomer in Form eines Gels aus Siloxanelastomer und einer Lipidphase verwendet wird, wobei der Gehalt des Siloxanelastomers in dem Gel 1 bis 80 Gew.-%, bevorzugt 0,1 bis 60 Gew.-% beträgt, jeweils bezogen auf das Gesamtgewicht des Gels.

Es ist vorteilhaft im Sinne der vorliegenden Erfindung, die Gesamtmenge der Siloxanelastomere (Aktivgehalt) aus dem Bereich von 0,01 bis 10 Gew.-%, vorteilhaft von 0,1 bis 5 Gew.-%, jeweils bezogen auf das Gesamtgewicht der Formulierung, zu wählen. Die erfindungsgemässen kosmetischen und dermatologischen Zubereitungen können Farbstoffe und/oder Farbpigmente enthalten, insbesondere wenn sie in Form von dekorativen Kosmetika vorliegen. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. Fe2O3, Fe3O4, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus dem Rowe Colour Index, 3. Auflage, Society of Dyers and Colourists, Bradford, England, 1971 zu wählen.

Sofern die erfindungsgemässen Formulierungen in Form von Produkten vorliegen, welche im Gesicht angewendet werden, ist es günstig, als Farbstoff eine oder mehrere Substanzen aus der folgenden Gruppe zu wählen: 2,4-Dihydroxyazobenzol, 1-(2'-Chlor-4'-nitro-1'- phenylazo)-2-hydroxynaphthalin, Ceresrot, 2-(Sulfo-1-naphthylazo)-1-naphthol-4-sulfosäure, Calciumsalz der 2-Hydroxy-1,2'-azonaphthalin-1'-sulfosäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4- Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfosäure, Aluminiumsalz der 4-(4-Sulfo-1-phenylazo)-1-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, rotes und schwarzes Eisenoxid (CIN: 77 491 (rot) und 77 499 (schwarz)), Eisenoxidhydrat (CIN: 77 492), Manganammoniumdiphosphat und Titandioxid.

Ferner vorteilhaft sind öllösliche Naturfarbstoffe, wie z.B. Paprikaextrakte, beta -Carotin oder Cochenille.

Vorteilhaft im Sinne der vorliegenden Erfindung sind ferner Formulierungen mit einem Gehalt an Perlglanzpigmenten. Bevorzugt sind insbesondere die im folgenden aufgelisteten Arten von Perlglanzpigmenten:
1. Natürliche Perlglanzpigmente, wie z.B.
   - "Fischsilber" (Guanin/Hypoxanthin-Mischkristalle aus Fischschuppen) und
   - "Perlmutt" (vermahlene Muschelschalen)
2. Monokristalline Perlglanzpigmente wie z. B. Bismuthoxychlorid (BiOCl)
3. Schicht-Substrat Pigmente: z.B. Glimmer/Metalloxid

Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteihaft ist z. B. das unter der CIN 77163 aufgelistete Glanzpigment.

Vorteilhaft sind ferner beispielsweise die folgenden Perlglanzpigmentarten auf Basis von Glimmer/Metalloxid:

| Gruppe | Belegung/Schichtdicke | Farbe |
|---|---|---|
| Silberweiße Perlglanzpigmente | TiO₂: 40-60 nm | silber |
| Interferenzpigmente | TiO₂: 60-80 nm | gelb |
| | TiO₂: 80-100 nm | rot |
| | TiO₂: 120-160 nm | grün |
| Farbglanzpigmente | Fe₂O₃ | bronze |
| | Fe₂O₃ | kupfer |
| | Fe₂O₃ | rot |
| | Fe₂O₃ | rotviolett |
| | Fe₂O₃ | rotgrün |
| | Fe₂O₃ | schwarz |
| Kombinationspigmente | TiO₂/Fe₂O₃ | Goldtöne |
| | TiO₂/Cr₂O₃ | grün |
| | TiO₂/Berliner Blau | tiefblau |
| | TiO₂/Carmin | rot |
| | | |

Besonders bevorzugt sind z.B. die von der Firma Merck unter den Handelsnamen Timiron, Colorona oder Dichrona erhältlichen Perlglanzpigmente.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate ausser Glimmer mit weiteren Metalloxiden beschichten, wie z.B. Silica und dergleichen mehr. Vorteilhaft sind z.B. mit TiO₂ und Fe₂O₃ beschichtete SiO₂-Partikel ("Ronaspheren"), die von der Firma Merck vertrieben werden und sich besonders für die optische Reduktion feiner Fältchen eignen.

Es kann darüber hinaus von Vorteil sein, gänzlich auf ein Substrat wie Glimmer zu verzichten. Besonders bevorzugt sind Eisenperlglanzpigmente, welche ohne die Verwendung von Glimmer hergestellt werden. Solche Pigmente sind z.B. unter dem Handelsnamen Sicopearl Kupfer 1000 bei der Firma BASF erhältlich.

Besonders vorteilhaft sind ferner auch Effektpigmente, welche unter der Handelsbezeichnung Metasomes Standard/Glitter in verschiedenen Farben (yello, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (CI) Nummern 19140, 77007, 77289, 77491) vor.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden. Die Gesamtmenge der Farbstoffe und farbgebenden Pigmente wird vorteilhaft aus dem Bereich von z.B. 0,1 Gew.-% bis 30 Gew.-%, vorzugsweise von 0,5 bis 15 Gew.-%, insbesondere von 1,0 bis 10 Gew.-% gewählt, jeweils bezogen auf das Gesamtgewicht der Zubereitungen.

Es ist auch vorteilhaft im Sinne der vorliegenden Erfindung, kosmetische und dermatologische Zubereitungen zu erstellen, deren hauptsächlicher Zweck nicht der Schutz vor Sonnenlicht ist, die aber dennoch einen Gehalt an weiteren UV-Schutzsubstanzen enthalten. So werden z. B. in Tagescremes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische und dermatologische Zubereitungen, die in der Form eines Sonnenschutzmittels vorliegen.

Dementsprechend enthalten die Zubereitungen im Sinne der vorliegenden Erfindung vorzugsweise mindestens eine weitere UV-A-, UV-B- und/oder Breitbandfiltersubstanz. Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.

Die Zubereitungen gemäss der vorliegenden Erfindung können ferner vorteilhaft auch in Form von sogenannten ölfreien kosmetischen oder dermatologischen Emulsionen vorliegen, welche eine Wasserphase und mindestens eine bei Raumtemperatur flüssige UV- Filtersubstanz als weitere Phase enthalten und welche insbesondere vorteilhaft auch frei von weiteren Ölkomponenten sein können.

Besonders vorteilhafte bei Raumtemperatur flüssige UV-Filtersubstanzen im Sinne der vorliegenden Erfindung sind Homomenthylsalicylat (INCI: Homosalate), 2-Ethylhexyl-2-cyano-3,3-diphenylacryiat (INCI: Octocrylene), 2-Ethylhexyl-2-hydroxybenzoat (2-Ethylhexylsalicylat, Octylsalicylat, INCI: Ethylhexyl Salicylate) und Ester der Zimtsäure, vorzugsweise 4-Methoxyzimtsäure(2-ethylhexyl)ester (2-Ethylhexyl-4-methoxycinnamat, INCI: Ethylhexyl Methoxycinnamate) und 4-Methoxyzimtsäureisopentylester (Isopentyl-4-methoxycinnamat, INCI: Isoamyl p-Methoxycinnamate), 3-(4-(2,2-bis Ethoxycarbonylvinyl)-phenoxy)propenyl)-methoxysiloxan/Dimethylsiloxan - Copolymer welches beispielsweise unter der Handelsbezeichnung Parsol® SLX bei Hoffmann La Roche erhältlich ist.

Bevorzugte anorganische Pigmente sind Metalloxide und/oder andere in Wasser schwerlösliche oder unlösliche Metallverbindungen, insbesondere Oxide des Titans (TiO₂), Zinks (ZnO), Eisens (z.B. Fe₂O₃), Zirkoniums (ZrO₂), Siliciums (SiO₂), Mangans (z.B. MnO), Aluminiums (Al₂O₃), Cers (z:B. Ce₂O₃), Mischoxide der entsprechenden Metalle sowie Abmischungen aus solchen Oxiden sowie das Sulfat des Bariums (BaSO₄).

Die Pigmente können vorteilhaft im Sinne der vorliegenden Erfindung auch in Form kommerziell erhältlicher öliger oder wässriger Vordispersionen zur Anwendung kommen. Diesen Vordispersionen können vorteilhaft Dispergierhilfsmittel und/oder Solubilisationsvermittler zugesetzt sein.

Die Pigmente können erfindungsgemäss vorteilhaft oberflächlich behandelt ("gecoatet") sein, wobei beispielsweise ein hydrophiler, amphiphiler oder hydrophober Charakter gebildet werden beziehungsweise erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)6, Natriummetaphosphat (NaPO₃)n, Siliciumdioxid (SiO₂) (auch: Silica, CAS-Nr.: 7631-86-9), oder Eisenoxid (Fe₂O₃). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen. Erfindungsgemäss geeignete Zinkoxidpartikel und Vordispersionen von Zinkoxidpartikeln sind unter folgenden Handelsbezeichnungen bei den aufgeführten Firmen erhältlich:

| Handelsname | Coating | Hersteller |
|---|---|---|
| Z-Cote HP 1 | 2 % Dimethicone | BASF |
| Z-Cote | / | BASF |
| ZnO NDM | 5 % Dimethicone | H & R |
| MZ-303S | 3 % Methicone | Tayca Corporation |
| MZ-505S | 5 % Methicone | Tayca Corporation |

Geeignete Titandioxidpartikel und Vordispersionen von Titandioxidpartikeln sind unter folgenden Handelsbezeichnungen bei den aufgeführten Firmen erhältlich:

| Handelsname | Coating | Hersteller |
|---|---|---|
| MT-100TV | Aluminiumhydroxid Stearinsäure | / Tayca Corporation |
| MT-100Z | Aluminiumhydroxid / Stearinsäure | Tayca Corporation |
| Eusolex T-2000 | Alumina / Simethicone | Merck KgaA |
| Titandioxid T805 (Uvinul TiO₂) | Octyltrimethoxysilan | Degussa |
| Tioveil AQ 10PG | Alumina / Silica | Solaveil / Uniquema |
| Eurolex T-aqua | Wasser / Alumina / Natriummetaphosphat | Merck |

Weitere vorteilhafte Pigmente sind Latexpartikel. Erfindungsgemäss vorteilhafte Latexpartikel sind die in den folgenden Schriften beschriebenen: US 5,663,213 bzw. EP 0 761 201. Besonders vorteilhafte Latexpartikel sind solche, welche aus Wasser und Styrol/Acrylat-Copolymeren gebildet werden und z. B. unter der Handelsbezeichnung "Alliance SunSphere" bei der Fa. Rohm & Haas erhältlich sind.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol TM 1789 und von Merck unter der Handelsbezeichnung Eusolex TM 9020 verkauft wird.

Vorteilhafte weitere UV-Filtersubstanzen im Sinne der vorliegenden Erfindung sind sulfonierte, wasserlösliche UV-Filter, wie z.B.:
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4bis-(2-benzimidazyl)-3,3'-5,5'-tetrasulfonsäure-bisnatriumsalz mit der INCI-Bezeichnung Disodium Phenyl Dibenzimidazol Tetrasulfonat (CAS-Nr.: 180898-37-7), welches beispielsweise unter der Handelsbezeichnung Neo Heliopan AP bei Haarmann & Reimer erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;
- 1,4-di(2-oxo-10-Sulfo-3-bornylidenmethyl)-Benzol (auch: 3,3'-(1,4-Phenylendimethylene)-bis-(7,7-dimethyl-2-oxobicyclo-[2.2.1]hept-1-ylmethan Sulfonsäure) und dessen Salze (besonders die entprechenden 10-Sulfatoverbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4- di(2-oxo-3bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Vorteilhafte UV-Filtersubstanzen im Sinne der vorliegenden Erfindung sind ferner Benzoxazol-Derivate, welche sich durch die folgende Strukturformel auszeichnen, 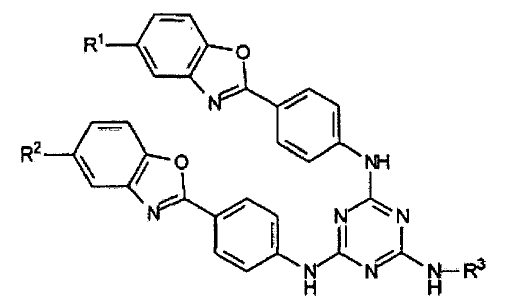 worin R¹, R² und R³ unabhängig voneinander gewählt werden aus der Gruppe der verzweigten oder unverzweigten, gesättigten oder ungesättigten Alkylreste mit 1 bis 10 Kohlenstoffatomen. Es ist erfindungsgemäss besonders vorteilhaft, die Reste R¹ und R² gleich zu wählen, insbesondere aus der Gruppe der verzweigten Alkylreste mit 3 bis 5 Kohlenstoffatomen. Es ist ferner besonders vorteilhaft im Sinne der vorliegenden Erfindung, wenn R³ einen unverzweigten oder verzweigten Alkylrest mit 8 Kohlenstoffatomen, insbesondere den 2-Ethylhexylrest darstellt.

Erfindungsgemäss besonders bevorzugtes Benzoxazol-Derivat ist das 2,4-bis-[5-1(dimethylpropyl)benzoxazol-2-yl-(4-phenyl)-imino]-6-(2-ethylhexyl)-imino-1,3,5-triazin mit der CAS Nr. 288254-16-0, welches sich durch die Strukturformel 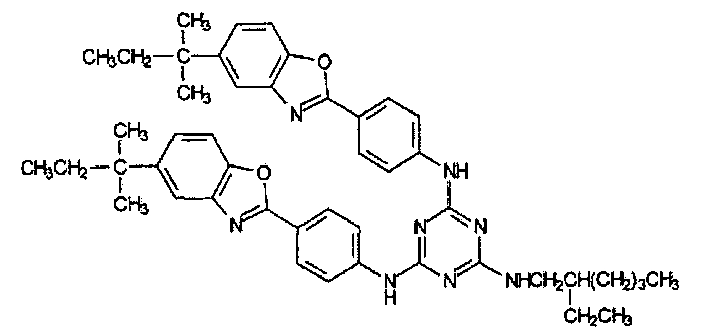 auszeichnet und bei 3V Sigma unter der Handelsbezeichnung Uvasorb TM K2A erhältlich ist.

Das oder die Benzoxazol-Derivate liegen vorteilhaft in gelöster Form in den erfindungsgemässen kosmetischen Zubereitungen vor. Es kann gegebenenfalls aber auch von Vorteil sein, wenn das oder die Benzoxazol-Derivate in pigmentärer, d.h. ungelöster Form - beispielsweise in Partikelgrössen von 10 nm bis zu 300 nm - vorliegen.

Vorteilhafte UV-Filtersubstanzen im Sinne der vorliegenden Erfindung sind ferner sogenannte Hydroxybenzophenone. Hydroxybenzophenone zeichnen sich durch die folgende Strukturformel aus: 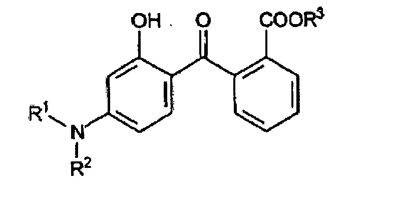 worin
- R¹ und R² unabhängig voneinander Wasserstoff, C₁-C₂₀₋Alkyl, C₃-C₁₀-Cycloalkyl oder C₃-C₁₀-Cycloalkenyl bedeuten, wobei die Substituenten R¹ und R² gemeinsam mit dem Stickstoffatom, an das sie gebunden sind, einen 5- oder 6-Ring bilden können und
- R³ einen C₁-C₂₀-Aikyl Rest bedeutet.
   Ein besonders vorteilhaftes Hydroxybenzophenon im Sinne der vorliegenden Erfindung ist der 2-(4'-Diethylamino-2'-hydoxybenzoyl)-benzoesäurehexylester (auch: Aminobenzophenon), welcher sich durch folgende Struktur auszeichnet: 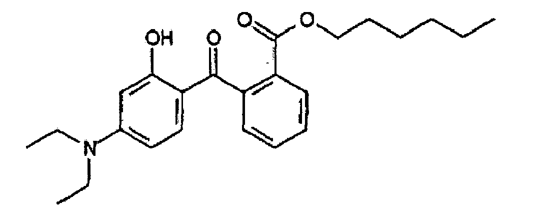 und unter dem Uvinul A Plus bei der Fa. BASF erhältlich ist.
   Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.
   Vorteilhafte Breitbandfilter oder UV-B-Filtersubstanzen sind beispielsweise Triazinderivate, wie z.B.
- 2,4-Bis- [4-(2-Ethyl-hexyloxy)-2-hydroxy]-phenyl-6-(4-methoxypheny()-1,3,5-triazin (INCI: Bis-Ethylhexyloxylphenol Methoxyphenyl Triazin), welches unter der Handelsbezeichnung Tinosorb TM S bei der CIBA-Chemikalien GmbH erhältlich ist;
- Dioctylbutylamidotriazon (INCI: Diethylhexyl Butamido Triazone), welches unter der Handelsbezeichnung UVASORB HEB bei Sigma 3 V erhältlich ist;
- 4,4',4"-(1,3,5-Triazin-2,4,6-triyltrümino)-tris-benzoesäure-tris(2-ethylhexylester), auch: 2,4,6-Tris-[anilino-(p-carbo-2'-ethyl-1'-hexyloxy)]-1,3,5-triazin (INCI: Ethylhexyl Triazone), welches von der BASF Aktiengesellschaft unter der Warenbezeichnung UVINUL TM T 150 vertrieben wird;
- 2-[4,6-Bis(2,4-dimethylphenyl)-1,3,5-triazin-2-yl]-5-(octyloxy)phenol (CAS Nr.: 2725- 22-6).

Ein vorteilhafter Breitbandfilter im Sinne der vorliegenden Erfindung ist auch das 2,2-Methylen-bis-(6-(2H-benzotriazol-2-yl)-4-(1,1,3,3-tetramethylbutyl)-phenol) (INCI: Methylene Bis-Benztriazolyl Tetramethylbutylphenol), welches z.B. unter der Handelsbezeichnung Tinosorb TM M bei der CIBA-Chemikalien GmbH erhältlich ist. Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane.

Die weiteren UV-Filtersubstanzen können öllöslich oder wasserlöslich sein. Vorteilhafte öllösliche Filtersubstanzen sind z.B.:
- 3-Benzylidencampher-Derivate, vorzugsweise 3-(4-Methylbenzyliden)campher, 3- Benzylidencampher;
- 4-Aminobenzoesäure-Derivate, vorzugsweise 4-(Dimethylamino)-benzoesäure(2- ethylhexyl)ester, 4-(Dimethylamino)benzoesäureamylester;
- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2- Hydroxy-4-methoxy-4'-methylbenzophenon, 2,2'-Dihydroxy-4-methoxybenzophenon sowie
- an Polymere gebundene UV-Filter.

Vorteilhafte wasserlösliche Filtersubstanzen sind z.B.: Sulfonsäure-Derivate des 3-Benzylidencamphers, wie z.B. 4-(2-Qxo-3-bornylidenmethyl)benzolsulfonsäure, 2-Methyl-5-(2-oxo-3-bornylidenmethyl)sulfonsäure und deren Salze.

Eine weitere erfindungsgemäss vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 T erhältlich ist.

Besonders vorteilhafte Zubereitungen im Sinne der vorliegenden Erfindung, die sich durch einen hohen beziehungsweise sehr hohen UV-A-Schutz auszeichnen, enthalten neben der oder den erfindungsgemässen Filtersubstanz(en) bevorzugt ferner weitere UV-A- und/oder Breitbandfilter, insbesondere Dibenzoylmethanderivate [beispielsweise das 4-(tert.-Butyl)-4'-methoxydibenzoylmethan] und/oder das 2,4-Bis- [4-(2-Ethylhexyloxy)-2-hydroxy]- phenyl-6-(4-methoxyphenyl)-1,3,5-triazin und/oder Phenylen-1,4-bis-(2-benzimidazyl)- 3,3'-5,5'-tetrasulfonsäure-bis-natriumsalz jeweils einzeln oder in beliebigen Kombinationen miteinander.

Die Liste der genannten UV-Filter, die im Sinne der vorliegenden Erfindung eingesetzt werden können, soll selbstverständlich nicht limitierend sein.

Vorteilhaft enthalten die Zubereitungen gemäss der vorliegenden Erfindung die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar beziehungsweise die Haut vor dem gesamten Bereich der ultravioletten Strahlung schützen.

Die Zubereitungen im Sinne der vorliegenden Erfindung können ferner vorteilhaft weitere Substanzen enthalten, die die Wasserfestigkeit der Produkte erhöhen.

Vorteilhaft sind beispielsweise wasserlösliche oder in Wasser dispergierbare Polyoxyethylen-Polyoxypropylen-Blockpolymere (CTFA-Bezeichnung: Polaxamere, CAS-Nr. 9003-11-6) mit folgender Struktur: 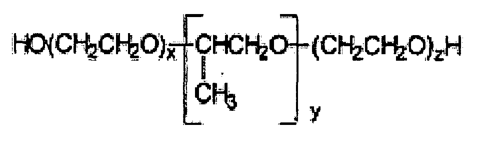 wobei x, y und z ganze Zahlen aus dem Bereich von 2 bis 130, insbesondere von 15 bis 100 darstellen und x und z gleich sind, aber unabhängig von y gewählt werden.

Unter diesen sind insbesondere Polaxamer 188 [mit x = 75, y = 30 und z = 75], welches unter der Handelsbezeichnung Lutrol F 68 (alt: Pluronic F 68) von der Firma BASF zu beziehen ist, das Polyxamer 185 [mit x = 19, y = 30 und z = 19] (Lubrajel WA von ISP), das Polyxamer 235 [mit x = 27, y = 39 und z = 27] (Pluronic F 85 von BASF) und/oder das Polyxamer 238 [mit x = 97, y = 39 und z = 97] (Pluronic F 88 von BASF) vorteilhaft zu verwenden.

Weitere vorteilhafte Substanzen, welche zur Steigerung der Wasserfestigkeit beitragen können, aber in die Ölphase der Zubereitungen gemäss der vorliegenden Erfindung eingearbeitet werden, sind bestimmte Wachskomponenten, wie acetyliertes Glykolstearat mit Tristearin (z.B. Unitwix von ISP mit der INCI: Acetylated Glycol Stearat and Tristearin), C18-36 Fettsäuretriglycerid (z.B. Syncrowax HGLC von der Fa. Crode GmbH mit der INCI: C18-36 Acid Triglyceride) und ferner die unter der Handelsbezeichnungen "Perfroma V 825" (synethisches Wachs) von New Phase Technologies erhältlichen Substanz sowie PEG-45 Dodecylglykolcopolymer (INCI: PEG-45 Dodecyl Glycol Copolymer), PEG-22 Dodecylglykolcopolymer (INCI: PEG-22 Dodecyl Glycol Copolymer), Methoxy PEG-22 Dodecylglykolcopolymer (INCI: Methoxy PEG-22 Dodecyl Glycol Copolymer) zu verwenden, welche von der Firma AKZO Nobel erhältlich sind.

Es ist besonders vorteilhaft im Sinne der vorliegenden Erfindung die erfindungsgemäss verwendeten Polymere mit einer oder mehreren der genannten Substanzen zu kombinieren, um die Wasserfestigkeit der Zubereitungen noch weiter zu verbessern.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele

### 1. Strukturmodifizierung

Die Strukturmodifizierung wird mittels einer Kugelmühle oder einer kontinuierlich arbeitenden Kugelmühle durchgeführt.
Die Nachvermahlung wird mittels einer Luftstrahlmühle, einer Zahnscheibenmühle oder einer Stiftmühle durchgeführt.

**Tabelle 2: Herstellung der strukturmodifizierten Titandioxide**

| | Oxid* | Strukturmodifizierung | Nachvermahlung** |
|---|---|---|---|
| Beispiel 1 | 1 | Ja | Nein |
| Beispiel 2 | 1 | Ja | ZM |
| Beispiel 3 | 1 | Ja | LS |
| Beispiel 4 | 1 | Ja | LS |
| Beispiel 5 | 2 | Ja | Nein |
| Beispiel 6 | 2 | Ja | ZM |
| Beispiel 7 | 2 | Ja | LS |
| Beispiel 8 | 2 | Ja | LS |

| | | | |
|---|---|---|---|
| *Oxid 1 = Aeroxide® TiO₂ P 25 | | | |
| Oxid 2 = Aeroxide® TiO₂ PF 2 | | | |
| **ZM = Zahnscheibenmühle | | | |
| LS = Luftstrahlmühle | | | |

**Tabelle 3: Physikalisch-chemische Daten der strukturmodifizierten Titandioxide**

| | Stampfdichte [g/l] | pH-Wert | Trocknungsverlust [%] | Glühverlust [%] | spez. Oberfläche n. BET [m²/g] |
|---|---|---|---|---|---|
| Beispiel 1 | 805 | 3,6 | 1,2 | 1,1 | 53 |
| Beispiel 2 | 735 | 3,6 | 1,0 | 0,7 | 52 |
| Beispiel 3 | 324 | 3,8 | 0,7 | 1,1 | 52 |
| Beispiel 4 | 446 | 3,8 | 0,6 | 1,2 | 50 |
| Beispiel 5 | 860 | 3,7 | 1,4 | 1,8 | 58 |
| Beispiel 6 | 764 | 3,6 | 1,4 | 1,3 | 58 |
| Beispiel 7 | 351 | 3,8 | 1,2 | 1,2 | 56 |
| Beispiel 8 | 299 | 3,8 | 1,1 | 1,3 | 59 |

### 2. Sonnenschutzformulierungen

### Dispersionsherstellung

278,25 g TEGOSOFT® TN werden in einem 500-ml-PE-Becher vorgelegt und 21,75 g des zu untersuchenden Titandioxid-Pulvers mit Hilfe eines Dissolvers (Pendraulik Typ LM34 Nr.29490, Scheibendurchmesser 6 cm) bei 470 U/min eingerührt und dann fünf Minuten bei 3000 U/min dispergiert.

Im Anschluß wird die Dispersion mit einem Ultra-Turrax-Rührer (Polytron PT3100, Dispergierwerkzeug PT-DA 3020/2 EC) zwei Minuten bei 15.000 U/min dispergiert.

Schließlich wird die Dispersion weitere fünf Minuten in einem wassergekühlten Behälter mit dem Ultra-Turrax-Rührer bei 15.000 U/min dispergiert, wobei nun das Dispergierwerkzeug PT-DA 3030-6060/3 EC verwendet wird.

### UV-Vis-Spektren (Transmission bei 380nm)

Die UV-Vis Spektren von 3 Gew.-%igen Dispersionen werden in einer zerlegbaren 10 µm-Quartzglas-Küvette mit einem UV-Vis-Spektralphotometer Specord 200 mit Photometerkugel (Firma Analytik Jena AG) gemessen. Dazu werden die oben beschriebenen öligen Dispersionen mit Tego-soft TN verdünnt.

Unter Rühren mit dem Dissolver (Pendraulik Typ LM34 Nr.29490, Scheiben-durchmesser 5 cm; 1000 - 4000 Umdrehungen / min) wird dann portionsweise AEROSIL® 200 zugegeben, um eine gelartige Masse herzustellen und das Oxid zu stabilisieren.

Nach der letzten AEROSIL-Zugabe muß mindestens 2 Minuten nachdispergiert werden um eine homogene Verteilung des AEROSIL zu gewährleisten. Als Ergebnis erhält man die Transmission (%) im Bereich von 290-500nm.

### Viskosität beziehungsweise Verdickungswirkung

Die Viskosität wird mit einem Brookfield Rheometer RVDV-III+cP ermittelt. Die Messung erfolgt in einem PE-Mischbecher (350ml) und dem RV-Spindelsatz, bei 10 U/min. Nach 1 Minute wird der Wert in mPas abgelesen.

**Tabelle 4: Charakterisierung von strukturmodifizierten AEROXIDE® TiO₂ P 25-Mustern**

| **Bezeichnung** | **Nachvermahlung** | **Stampfdichte (g/l)** | **Transmission bei 380 nm (%)** | **Viskosität (mPas) (Spindel-Typ)** |
|---|---|---|---|---|
| Beispiel 1 | - | 805 | 34 | 80 (1) |
| Beispiel 2 | ZM | 735 | 45 | 63 (1) |
| Vergleichsbeispiel AEROXIDE TiO₂ P25 | - | 282 | 22 | 520 (2) |

Vorteile der erfindungsgemäßen Produkte aus Beispiel 1 und 2 gegenüber dem Vergleichsbeispiel mit AEROXIDE TiO₂ P 25 (nicht strukturmodifiziert) sind
- eine wesentlich erhöhte Stampfdichte. Dies bedeutet eine vereinfachte Handhabung und Verarbeitung, geringeren Transport- und Lageraufwand sowie eine reduzierte Staubentwicklung.
- Eine deutliche erhöhte Transmission bei 380 nm. Dies bedeutet eine erhöhte Transparenz beziehungsweise reduziertes Weißeln damit hergestellter Sonnenschutzformulierungen.
- Eine erhebliche reduzierte Viskosität beziehungsweise Verdickungswirkung. Dies ermöglicht die Herstellung von Dispersionen beziehungsweise Sonnenschutzformulierungen mit höherem Titandioxidgehalt und damit einem höheren Lichtschutzfaktor.

**Sonnenschutzformulierung 1**

| **A.** | **%** | **Bestandteil** | |
|---|---|---|---|
| | 3.00 | Isopropyl Myristate | Isopropyl Myristate |
| | 8.00 | Jojoba Oil | Simmondsia Chinensis (Jojoba) seed oil |
| | 4.00 | Uvinul® MC 80 | Octyl Methoxycinnamate |
| | 1.00 | Abil® 350 | Dimethicone |
| | 6.00 | Cremophor® WO 7 | PEG-7 Hydrogenated Castor Oil |
| | 2.00 | Ganex® V 216 | PVP/Hexadecene Copolymer |
| | 2.00 | Elfacos® ST 9 | PEG-45/Ddecyl Glycol Copolymer |
| | 2.00 | Uvinul® MBC 95 | 4-Methylbenzylidene Camphor |
| **B** | 3.00 | Feinteiliges Titandioxid | Titanium Dioxide |
| | 5.00 | Z-Cote® HP 1 | Zinc Oxide (und) Dimethicone |
| **C** | 1.00 | Magnesium sulfate-7-hydrate | Magnesium Sulfate |
| | 5.00 | Glycerin 87 % | Glycerin |
| | 0.20 | Edeta® BD | Disodium EDTA |
| | 0.30 | Germoll® 115 | Imidazolidinyl Urea |
| | 57.0 0 | Water dem. | Wasser |
| | q.s. | Perfume | |
| | 0.50 | Euxyl® K3000 | Phenoxyethanol, Methylparaben, Butylparaben, Ethylparaben, Propylparaben, Isobutyl paraben |

Die Phase A wird auf 80 °C erhitzt, die Phase B wird hinzugefügt und für 3 Minuten homogenisiert.

Die Phase C wird auf 80 °C erhitzt und in die Mischung aus den Phasen A und B unter Homogenisieren eingerührt.

**Sonnenschutzformulierung 2**

| **A.** | **%** | **Bestandteil** | |
|---|---|---|---|
| | 6.00 | Cremophor® WO 7 | PEG-7 Hydrogenated Castor Oil |
| | 2.00 | Elfacos® ST 9 | PEG-45/Dodecyl GlycolCopolymer |
| | 3.00 | Isopropyl Myristate | Isopropyl Myristate |
| | 8.00 | Jojoba Oil | Jojoba (Buxus Chinensis) Oil |
| | 4.00 | Uvinul® MC 80 | Octyl Methoxycinnamate |
| | 2.00 | Uvinul® MBC 95 | 4-Methylbenzylidene Camphor |
| | 3.00 | 1. Feinteiliges Titandioxid | Titanium Dioxide |
| | 1.00 | Abil® 350 | Dimethicone |
| | 5.00 | Z-Cote® HP 1 | Zinc Oxide, Dimethicone |
| **B** | 0.20 | Edeta® BD | Disodium EDTA |
| | 5.00 | Glycerin 87 % | Glycerin |
| | q.s. | Preservative | |
| | 60.8 0 | Water dem. | Aqua dem. |
| **C** | q.s. | Perfume | |

Die Phase A und B werden separat auf 85 °C erhitzt. Anschließend wird die Phase B in die Phase A eingerührt und homogenisiert. Die Mischung wird auf 40 °C abgekühlt, die Phase C hinzugegeben und anschließend erneut homogenisiert.

**Ergebnisse**

| | Formulierung 1 | Formulierung 2 |
|---|---|---|
| | Transparenz auf der Haut | Transparenz auf der Haut |
| Vergleichsbeispiel AEROXIDE TiO2 T805 | nicht befriedigend | nicht befriedigend |
| Beispiel 2 | befriedigend | befriedigend |

## Patentansprüche

1. Pyrogen hergestellte, strukturmodifizierte Titandioxide beziehungsweise pyrogen hergestellte, strukturmodifizierte Titandioxid-Mischoxide.

2. Verfahren zur Herstellung der pyrogen hergestellten, strukturmodifizierten Titandioxide beziehungsweise der pyrogen hergestellten, strukturmodifizierten Titandioxid-Mischoxide gemäß Anspruch 1, **dadurch gekennzeichnet, daß** man das pyrogen hergestellte Titandioxid beziehungsweise das pyrogen hergestellte, strukturmodifizierte Titandioxid-Mischoxid mit einer Kugelmühle behandelt und gegebenenfalls nachvermahlt.

3. Verwendung der pyrogen hergestellten, strukturmodifizierten Titandioxide beziehungsweise derpyrogen hergestellten, strukturmodifizierten Titandioxid-Mischoxide gemäß Anspruch 1 zur Herstellung von Sonnenschutzformulierungen.

4. Sonnenschutzformulierungen, **dadurch gekennzeichnet, daß** sie pyrogen hergestellte, strukturmodifizierte Titandioxide beziehungsweise pyrogen hergestellte, strukturmodifizierte Titandioxid-Mischungen gemäß Anspruch 1 enthalten.
